# EUROPEAN PATENT SPECIFICATION

(11) **EP 1 928 321 B1**
(45) Date of publication and mention of the grant of the patent: **01.09.2010**
(21) Application number: 06811000.6
(22) Date of filing: 26.09.2006
(51) Int. Cl.: A61B 8/12, G01N 29/28, G10K 11/02

(54) **ULTRASONIC PROBE**
ULTRASCHALLSONDE
SONDE ULTRASONORE

(30) Priority: 30.09.2005 JP 2005286116
(43) Date of publication of application: 11.06.2008
(73) Proprietor: FUJIFILM Corporation, Minato-ku Tokyo 106-8620 (JP); Fujinon Corporation, Saitama-shi Saitama 331-9624 (JP)
(72) Inventor: KUNIYASU, Toshiaki, Ashigarakami-gun, Kanagawa 258-8577 (JP); TANAKA, Toshizumi, Saitama-shi, Saitama 331-9624 (JP); ITOI, Hiromu, Saitama-shi, Saitama 331-9624 (JP)
(74) Representative: Höhfeld, Jochen
(86) International application number: PCT/JP2006/319645
(87) International publication number: WO 2007/037464

(56) References cited:
- JP-A- 2001 299 748
- JP-A- 2005 146 251
- US-A- 6 039 695
- 'MORESCO_WHITEP-70 technical information' MATSUMURA OIL RESEARCH CORP., [Online] XP003012403 Retrieved from the Internet: <URL:http://www.moresco.co.jp/moresco_web/c ontents/products/material_section/info/para ffin/inf_P-70.pdf>

## Description

### Technical Field

The present invention generally relates to an ultrasonic generator used in ultrasonic probes, and more particularly to an ultrasonic generator with improved ultrasonic transfer characteristics.

### Background Art

Some medical electronic endoscopes have both a CCD camera for observation of an organ surface and an ultrasonic probe for observation inside the tissues of the organ. The ultrasonic probe sends ultrasonic waves into a biological tissue of interest and receives echoes of the ultrasonic waves. The received echoes of the ultrasonic waves bear tomographic information of the biological tissue, and it is possible to produce a tomographic image of the biological tissue based on these echoes. The combination of the ultrasonic image inside the biological tissue by the ultrasonic probe and an optical image of the biological tissue by the CCD camera enables accurate diagnosis as to existence of a disease and progress of the disease.

The ultrasonic prove has one or more ultrasonic transducers as a source of ultrasonic wave. The ultrasonic transducer is composed of a piezoelectric element, and typically contained in a cavity filled with ultrasonic transmission fluid medium so that the ultrasonic waves are effectively transmitted to the target body. One variety of such transmission fluid medium is liquid paraffin, a kind of hydrocarbon oils (see, for example, US 6 039 695 and Japanese patent laid-open publication No. 05-176931). Also, Japanese laid-open publication No. 2001-299748 discloses an ultrasonic probe using the liquid paraffin having a kinematic viscosity of less than 20mm²/s. This liquid paraffin provides better transfer efficiency to high frequency components of the ultrasonic waves.

It becomes known, however, that the ultrasonic transfer characteristics of the liquid paraffin depends on molecular weight distribution of the paraffin molecules. It is therefore understood that the composition of the liquid paraffin must be considered for a good ultrasonic transfer characteristics.

In view of the foregoing, an object of the present invention is to provide an ultrasonic generator which incorporates the liquid paraffin with an excellent ultrasonic transfer characteristics.

### Disclosure of Invention

In order to achieve the above object and other objects, an ultrasonic generator according to the present invention includes liquid paraffin, as an ultrasonic transmission medium, whose maximum molecular weight M1 is 520 ≤ M1 ≤ 1400. Contained in a space filled with this liquid paraffin is an ultrasonic transducer which generates ultrasonic waves.

In addition, the liquid paraffin has a peak top molecular weight M2, where the number of molecules is greatest, of 230 ≤ M2 ≤ 380.

Furthermore, the liquid paraffin has a kinematic viscosity S of 4 mm²/s ≤ S ≤ 12.5 mm²/s.

It is preferable that the liquid paraffin has the maximum molecular weight M1 of 1300 ≤ M1 ≤ 1400.

It is also preferable that the liquid paraffin has the peak top molecular weight M2 of 320 ≤ M2 ≤ 380.

It is further preferable that the liquid paraffin has the kinematic viscosity S of 9 mm²/s ≤ S ≤ 12.5 mm²/s.

According to the present invention, the liquid paraffin is improved in the ultrasonic transfer characteristics. This enables deep penetration of the ultrasonic waves into the biological tissue and high receiver sensitivity across a broad frequency range. It is therefore possible to obtain high definition, high contrast ultrasonic images of deep part of a biological tissue.

### Brief Description of Drawings

Figure 1 is a schematic view of an electronic endoscope having an ultrasonic probe according to the present invention;
Figure 2 is a cross sectional view of the ultrasonic probe;
Figures 3A to 3D are graphs showing molecular weight distribution of four types of liquid paraffin;
Figure 4 is a table showing representative values for kinematic viscosity at different temperatures and the molecular weight distribution of the four types of liquid paraffin;
Figure 5 is a graph showing the kinematic viscosity at 40° C and penetration of the four types of liquid paraffin; and
Figure 6 is a graph showing receiver sensitivities of an optimal liquid paraffin and a conventional liquid paraffin at each frequency.

### Best Mode for Carrying Out the Invention

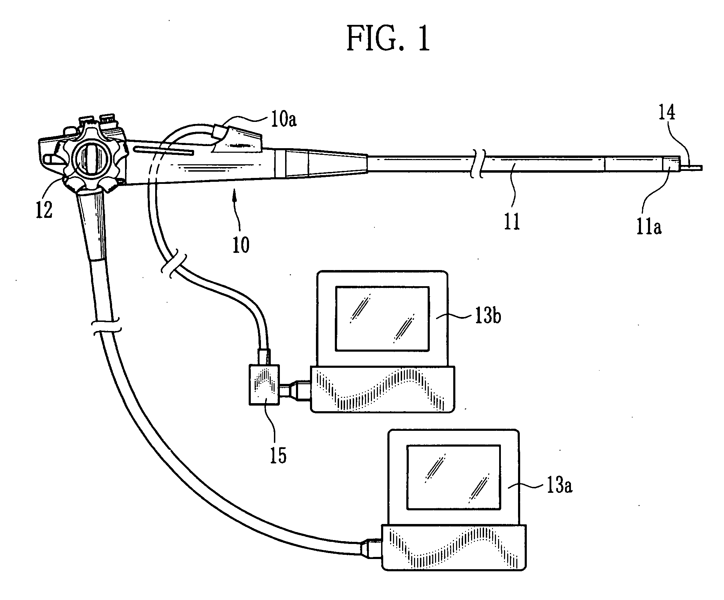

Referring to FIG.1, an electronic endoscope 10 has a flexible insertion section 11 to be inserted into a living body and an operating section 12 for operation of the insertion section 11. A distal end 11a of the insertion section 11 has a CCD camera (not shown). The electronic endoscope 10 is connected to monitors 13a and 13b, and the images captured with the CCD camera are displayed on the monitor 13a. At the time of an ultrasonic diagnosis, an ultrasonic probe 14 is inserted into an instrument inlet port 10a. The ultrasonic probe 14 passes through a forceps channel (not shown) running inside the insertion section 11, and projects outwardly from a forceps opening provided on the distal end 11a. The ultrasonic images obtained with the ultrasonic probe 14 are displayed on the monitor 13b.

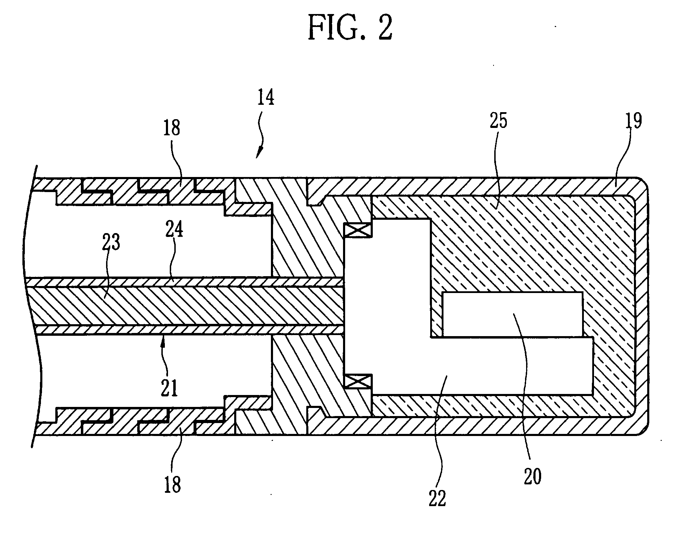

As shown in FIG.2, the ultrasonic probe 14 includes a flexible pipe section 18 constituted of a plurality of sheaths 18a interlocked with one another and a polyethylene cap 19 attached to the front end of the pipe section 18. Enclosed inside the cap 19 is an ultrasonic transducer 20 which generates ultrasonic waves and receives the echoes of the ultrasonic waves that reflected off the organs. The ultrasonic transducer 20 is mounted on a base 22, which is connected to a control cable 21. The control cable 21 is composed of a flexible shaft 23 and a flexible tube 24 to enclose the flexible shaft 23. One end of the flexible shaft 23 is connected to the base 22, and the other end thereof is connected to a translator 15 (see, FIG.1). The translator 15 has a built-in motor, and the drive of this motor leads the flexible shaft 23 to rotate around its axial direction. Accordingly, the base 22 rotates together with the ultrasonic transducer 20 on the flexible shaft 23. Rotating the ultrasonic transducer 20 enables radial scanning of the biological tissues with the ultrasonic waves to produce the ultrasonic images. It is understood that the flexible shaft 23 houses a plurality of electric wires for transmittal of drive signals to the ultrasonic transducer 20 and echo signals from the ultrasonic transducer 20.

Inside the cap 19 to contain the ultrasonic transducer 20 is filled with liquid paraffin 25 serving as an ultrasonic transmission medium. The liquid paraffin 25 improves transfer efficiency of the ultrasonic waves generated by the ultrasonic transducer 20 and the echoes of the ultrasonic waves reflected off the biological tissue. Also, the liquid paraffin 25 serves as a lubricant to allow smooth rotation of the ultrasonic transducer 20. The liquid paraffin 25, a kind of hydrocarbon oil, is superior in lubricity and electrical insulation property, and harmless to the living body.

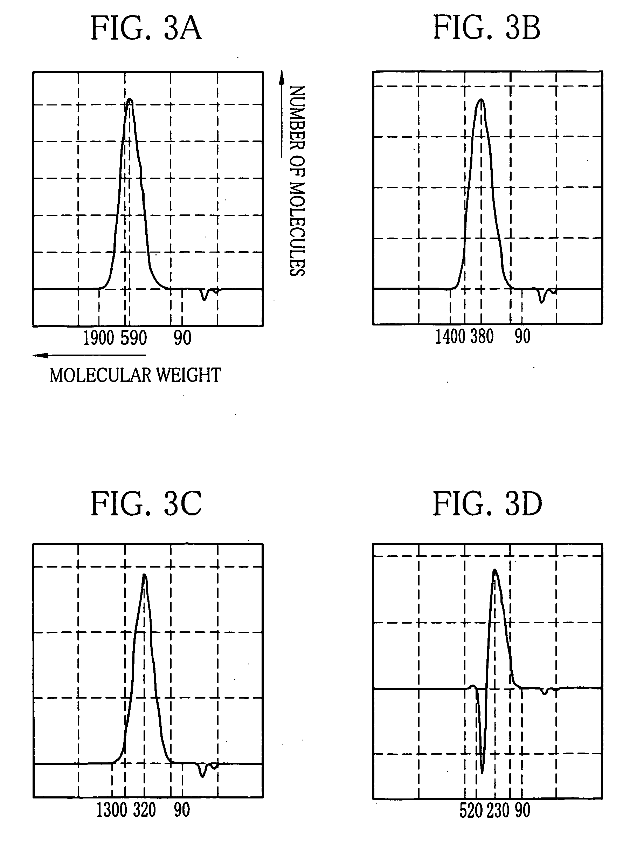

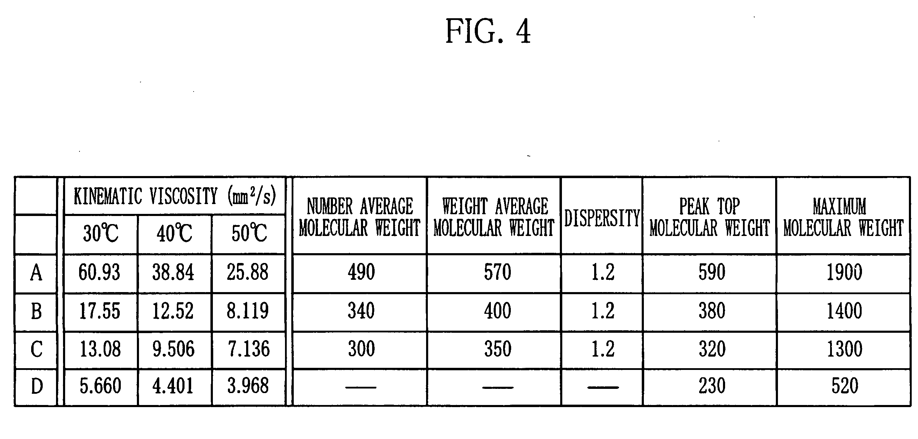

Next, the ultrasonic transfer characteristics was compared between four types of liquid paraffin A to D (from Wako pure chemical industries, ltd) having different compositions. The liquid paraffin A is the one used in conventional ultrasonic probes. The liquid paraffin B has a density of 0.825 to 0.850g/ml. The liquid paraffin C has a density of 0.815 to 0.840g/ml. The liquid paraffin D has a density of 0.795 to 0.830g/ml. The molecular weight distribution of each liquid paraffin was measured, and the results of the measurement are shown on graphs of FIGS.3A to 3D where the vertical axis represents the number of paraffin molecules and the horizontal axis represents the molecular weight of paraffin. In addition, a table of FIG. 4 shows kinematic viscosity of these liquid paraffin measured at different temperatures and representative values of the molecular weight distribution.

The molecular weight was measured by a gel permeation chromatography method (GPC method). Used in the measurement were LC-10 from Shimadzu corporation as the analytical instrument, PLgel MIXD-E columns, chloroform (1ml/min) as the elution solution, a differential refractive index detector, and polystyrene as a reference material. The values of each molecular weight are expressed in terms of polyethylene equivalent. The kinematic viscosity was measured under JIS K2283 (crude petroleum and petroleum products - Determination of kinematic viscosity and calculation of viscosity index from kinematic viscosity).

As shown in FIGS.3A to 3B, the liquid paraffin A used in the conventional ultrasonic probes showed a minimum molecular weight of approximately 90 and a maximum molecular weight of approximately 1900. Additionally, a peak top molecular weight, where the molecule number is largest, was 590, and a number averaged molecular weight, or the central value was 490. A weight averaged molecular weight was 570. The liquid paraffin B showed the minimum molecular weight of approximately 90 and the maximum molecular weight of approximately 1400. The peak top molecular weight was 380, and the number averaged molecular weight was 340. The weight averaged molecular weight was 400. The liquid paraffin C showed the minimum molecular weight of approximately 90 and the maximum molecular weight of approximately 1300. The peak top molecular weight was 320, and the number averaged molecular weight was 300. The weight averaged molecular weight was 350. The liquid paraffin D showed the minimum molecular weight of approximately 90 and the maximum molecular weight of approximately 1900. The peak top molecular weight was 230 while the number averaged molecular weight and the weight averaged molecular weight were immeasurable.

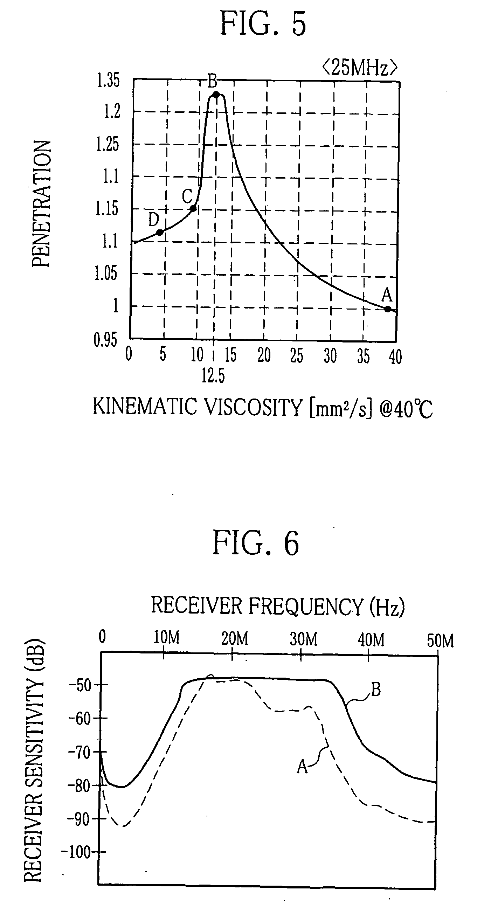

Recognized in FIG. 4 is that the liquid paraffin with higher values of the maximum, average, and peak top molecular weights has higher kinematic viscosity whereas the liquid paraffin with lower values of the maximum, average, and peak top molecular weights has lower kinematic viscosity. Next, the ultrasonic probes having one of the liquid paraffin A to D were prepared to measure a penetration of a 25MHz ultrasonic wave from these ultrasonic probes into a biological tissue. The measurement result is shown in FIG.5.

As shown in FIG.5, the liquid paraffin B showed by far greater penetration than the other liquid paraffin. This means that the liquid paraffin B enables to produce tomographic images of deeper part of a biological tissue. While being inferior to the liquid paraffin B, the liquid paraffin C and D were superior to the liquid paraffin A, proving to achieve a practically admissible level of penetration. These results lead to arrive the conclusion that an ever better penetration of the ultrasonic waves is achieved by the liquid paraffin having the kinematic viscosity S of 4 mm²/s ≤ S ≤ 12.5 mm²/s at 40°C, i.e., about the temperature of body. Specifically, the penetration becomes excellent when the kinematic viscosity S is 9 mm²/s ≤ S ≤ 12.5 mm²/s at 40°C.

FIG.6 has a graph showing a measurement result of receiver sensitivity at each frequency. The dashed line represents the conventional liquid paraffin A, and the solid line represents the liquid paraffin B which showed the best penetration among the others. The liquid paraffin A shows its best receiver sensitivity of -50dB at around 20MHz and the receiver sensitivity of -60dB at around 15MHz and 30MHz. The liquid paraffin B, on the other hand, shows good receiver sensitivity of -50dB across a broad frequency range stretching from around 12MHz to around 35MHz, proving to have by far greater receiver sensitivity than the liquid paraffin A. This means that the ultrasonic probes with the liquid paraffin B are able to produce high definition, high contrast ultrasonic images. Furthermore, the resolving power becomes excellent with the liquid paraffin B.

Based on the results of the above experiment, it is discovered that the ever better ultrasonic transfer characteristics can be obtained with the liquid paraffin having the maximum molecular weight of less than 1900 and in the range of 520 to 1400, and with the peak top molecular weight of less than 590 and in the range of 230 to 380. If the liquid paraffin is conditioned to have the maximum molecular weight of in the range of 1300 to 1400 and the peak top molecular weight of in the range of 320 to 380, an excellent or good ultrasonic transfer characteristics such as of the liquid paraffin B or C can be obtained.

The ultrasonic generator of the present invention is not limited to the medical electronic endoscopes having a CCD camera, but applicable to medical ultrasonic apparatuses of external type. Furthermore, the ultrasonic generator of the present invention is not limited to the medical endoscopes, but applicable to the industrial-use inspection apparatuses which work in the same frequency range of ultrasonic wave as the medical endoscopes.

### Industrial Applicability

The ultrasonic generator of the present invention is suitably applied to medical diagnosis apparatuses and industrial-use inspection apparatuses.

## Claims

1. An ultrasonic generator comprising:
an ultrasonic transducer for generating ultrasonic waves;
a cap for enclosing said ultrasonic transducer; and
liquid paraffin filled in said cap and having a maximum molecular weight M1 of 520 ≤ M1 ≤ 1400.

2. An ultrasonic generator described in claim 1, wherein said liquid paraffin has a peak top molecular weight M2 with a largest molecule number of 230 s M2 s 380.

3. An ultrasonic generator described in claim 1, wherein said liquid paraffin has a kinematic viscosity S of 4 mm²/s ≤ S ≤ 12.5 mm²/s at 40°C.

4. An ultrasonic generator described in claim 1, wherein said maximum molecular weight M1 is 1300 ≤ M1 ≤ 1400.

5. An ultrasonic generator described in claim 2, wherein said peak top molecular weight M2 is 320 s M2 ≤ 380.

6. An ultrasonic generator described in claim 3, wherein said kinematic viscosity S is 9 mm²/s ≤ S ≤ 12.5 mm²/s at 40°C.

## Patentansprüche

1. Ultraschallgenerator, umfassend:
einen Ultraschallwandler zum Erzeugen von Ultraschallwellen;
eine Kappe zum Einschließen des Ultraschallwandlers; und
Flüssigparaffin, welches in die Kappe eingefüllt ist und ein maximales Molekulargewicht M1 von 520 ≤ M1 ≤ 1400 aufweist.

2. Ultraschallgenerator nach Anspruch 1, bei dem das Flüssigparaffin ein Spitzen-Molekulargewicht M2 mit einer größten Molekülzahl von 230 ≤ M2 ≤ 380 besitzt.

3. Ultraschallgenerator nach Anspruch 1, bei dem das Flüssigparaffin eine kinematische Viskosität S von 4 mm²/s ≤ S ≤ 12,5 mm²/s bei 40°C besitzt.

4. Ultraschallgenerator nach Anspruch 1, bei dem das maximale Molekulargewicht M1 1300 ≤ M1 ≤ 1400 beträgt.

5. Ultraschallgenerator nach Anspruch 2, bei dem das Spitzen-Molekulargewicht M2 320 ≤ M2 ≤ 380 beträgt.

6. Ultraschallgenerator nach Anspruch 3, bei dem die kinematische Viskosität S 9 mm²/s ≤ S ≤ 12,5 mm²/s bei 40°C beträgt.

## Revendications

1. Générateur d'ultrasons comprenant :
un transducteur ultrasonique pour générer des ondes ultrasonores ;
un capuchon pour enfermer ledit transducteur ultrasonique ; et
de la paraffine liquide déversée dans ledit capuchon et ayant un poids moléculaire maximum M1 de 520 ≤ M1 ≤ 1400.

2. Générateur d'ultrasons selon la revendication 1, dans lequel ladite paraffine liquide a un poids moléculaire supérieur de pic M2 avec le plus grand nombre de molécules de 230 ≤ M2 ≤ 380.

3. Générateur d'ultrasons selon la revendication 1, dans lequel ladite paraffine liquide a une viscosité cinétique S de 4 mm²/s ≤ S ≤ 12,5 mm²/s à 40°C.

4. Générateur d'ultrasons selon la revendication 1, dans lequel ledit poids moléculaire maximum M1 est de 1300 ≤ M1 ≤ 1400.

5. Générateur d'ultrasons selon la revendication 2, dans lequel ledit poids moléculaire supérieur de pic M2 est de 320 ≤ M2 ≤ 380.

6. Générateur d'ultrasons selon la revendication 3, dans lequel ladite viscosité cinétique S est de 9 mm²/s ≤ S ≤ 12,5 mm²/s à 40°C.
